# EUROPEAN PATENT APPLICATION

(11) **EP 4 613 869 A1**
(43) Date of publication of application: **10.09.2025**
(21) Application number: 23885779.1
(22) Date of filing: 31.10.2023
(51) Int. Cl.: C12P 5/02, C12N 1/00, C12M 1/36, C12M 1/38

(54) **METHOD FOR PRODUCING METHANE, CONTROL DEVICE, AND METHANE PRODUCTION SYSTEM**

(30) Priority: 31.10.2022 JP 2022175065
(71) Applicant: Yokogawa Electric Corporation, Tokyo 180-8750 (JP)
(72) Inventor: KAWANO, Makoto, Musashino-shi, Tokyo 180-8750 (JP); KIMURA, Hiroyuki, Shizuoka-Shi, Shizuoka 422-8529 (JP)
(74) Representative: Stöckeler, Ferdinand
(86) International application number: PCT/JP2023/039329
(87) International publication number: WO 2024/096025

(57) **Abstract**

A method of producing methane using a methanogen, as well as a control device and a methane production system useable in this method, that achieve an excellent methane production capability continuously even under oxygen (O₂) contamination conditions is provided. The method of producing methane includes co-culturing a methanogen and a facultative anaerobic bacterium to produce methane, and the control device and methane production system are useable in this method.

## Description

### TECHNICAL FIELD

The present disclosure relates to a method of producing methane, a control device, and a methane production system.

### BACKGROUND

In order to meet the recent demand for clean energy, the use of methane as an energy source and the generation of methane using methanogens have been considered. For example, Patent Literature (PTL) 1 discloses a biogas generator including a hydrogen fermentation unit that anaerobically ferments organic liquid waste using hydrogen-producing bacteria to produce hydrogen and methane raw organic material, and a methane fermentation unit that anaerobically ferments the methane raw organic material using a methanogen to produce methane. PTL 2 discloses a methane production method for obtaining methane gas by co-culturing purple non-sulfur bacteria and a hydrogenotrophic methanogen, which can be co-cultured by a medium that does not contain ammonium salts and contains L-cysteine hydrochloride as a reducing agent, in the presence of a carbon dioxide-containing gas.

### CITATION LIST

### Patent Literature

PTL 1: JP 2001-149983 A
PTL 2: JP 2013-192547 A

### SUMMARY

### (Technical Problem)

Methanogens that produce methane gas are strictly anaerobic microorganisms, and the presence of oxygen (O₂) in the reaction vessel leads to a decrease in activity or death of the methanogens. When liquid or gas is supplied from outside to the reaction vessel for methanogenesis, there is a risk of contamination by oxygen as an impurity, but neither reference discloses a method related to measures against oxygen contamination, such as oxygen removal. If no measures are taken to prevent oxygen contamination, the expected methane production may not be achieved due to the effect of mixed-in oxygen.

It would be helpful to provide a method of producing methane using a methanogen, as well as a control device and a methane production system useable in this method, that continuously achieve an excellent methane production capability even under oxygen contamination conditions.

### (Solution to Problem)

A method of producing methane according to several embodiments includes co-culturing a methanogen and a facultative anaerobic bacterium to produce methane. This achieves continuous production of methane with an excellent methane production capability even under oxygen contamination conditions.

An embodiment may further include
monitoring a culture condition of a culture system;
determining whether the monitored culture condition is outside a set threshold range; and
adjusting a culture condition to increase oxygen consumption activity of the facultative anaerobic bacterium in a case in which the monitored culture condition is outside the set threshold range.

In an embodiment, the monitored culture condition may be the oxygen concentration, and a controlled culture condition may be the culture temperature.

In an embodiment, the monitored culture condition and a controlled culture condition may be culture temperature, pH of a culture medium, oxidation-reduction potential of the culture medium, or pressure in a reaction vessel.

A control device according to several embodiments is a control device for controlling culture conditions for co-culturing a methanogen and a facultative anaerobic bacterium, the control device including:
a sensor configured to monitor a culture condition of a culture system; and
a controller configured to determine whether the monitored culture condition is outside a set threshold range and control a culture condition to increase oxygen consumption activity of the facultative anaerobic bacterium in a case in which the monitored culture condition is outside the set threshold range.

This achieves continuous production of methane with an excellent methane production capability even under oxygen contamination conditions.

In an embodiment, the monitored culture condition may be oxygen concentration, and the controlled culture condition may be culture temperature.

In an embodiment, the monitored culture condition and the controlled culture condition may be culture temperature, pH of a culture medium, oxidation-reduction potential of the culture medium, or pressure in a reaction vessel.

A methane production system according to several embodiments includes:
the above-described control device;
a reaction vessel for co-culturing a methanogen and a facultative anaerobic bacterium; and
a mechanism configured to adjust a culture condition for co-culturing the methanogen and the facultative anaerobic bacterium, wherein
adjustment of the culture condition in the mechanism configured to adjust the culture condition is controlled by the control device.

This achieves continuous production of methane with an excellent methane production capability even under oxygen contamination conditions.

### (Advantageous Effect)

According to the present disclosure, a method of producing methane using a methanogen, as well as a control device and a methane production system useable in this method, that continuously achieve an excellent methane production capability even under oxygen (O₂) contamination conditions can be provided.

### BRIEF DESCRIPTION OF THE DRAWINGS

In the accompanying drawings:
FIG. 1 is a conceptual diagram illustrating an example of a methane production system of the present disclosure;
FIG. 2 is a graph illustrating the change over time in the concentration of methane in the gas phase in a reaction vessel in Example 1, where the arrows indicate the time of gas replacement in the reaction vessel;
FIG. 3 is a graph illustrating the change over time in the concentration of methane in the gas phase in a reaction vessel in Reference Example 1, where the arrows indicate the time of gas replacement in the reaction vessel; and
FIG. 4 is a graph illustrating the change over time in the concentration of methane in the gas phase in a reaction vessel in Comparative Example 1, where the arrows indicate the time of gas replacement in the reaction vessel.

### DETAILED DESCRIPTION

### (Method Of Producing Methane)

The method of producing methane according to the present disclosure includes co-culturing a methanogen and a facultative anaerobic bacterium to produce methane. In the method of producing methane of the present disclosure, methane can be obtained by supplying raw material for methanogenesis as a gas or a component of the culture medium and collecting the gas in the culture system containing the produced methane. By co-culturing a methanogen and a facultative anaerobic bacterium, the facultative anaerobic bacterium consumes oxygen (O₂) that has mixed in, thereby maintaining a low-oxygen condition in the culture system and maintaining good methanogenic activity of the methanogen. This achieves continuous production of methane with an excellent methane production capability even under oxygen contamination conditions.

The method of producing methane of the present disclosure may also include monitoring a culture condition of a culture system, determining whether the monitored culture condition is outside a set threshold range, and adjusting a culture condition to increase oxygen consumption activity of the facultative anaerobic bacterium in a case in which the monitored culture condition is outside the set threshold range. In the method of producing methane of the present disclosure, the monitored culture condition may be the oxygen concentration, and a controlled culture condition may be the culture temperature. Alternatively, in the method of producing methane of the present disclosure, the monitored culture condition and the controlled culture condition may be the same, and examples thereof include the culture temperature, the pH of the culture medium, the oxidation-reduction potential (ORP) of the culture medium, and the pressure in the reaction vessel. By adjusting the culture conditions, a good methanogenic activity of the methanogen can be maintained, thereby achieving continuous production of methane with an excellent methane production capability even under oxygen contamination conditions. For example, by monitoring the oxygen concentration and adjusting the culture temperature, a low-oxygen condition can be maintained in the culture system to maintain a good methanogenic activity of the methanogen, thereby achieving continuous production of methane with an excellent methane production capability even under oxygen contamination conditions.

In the method of producing methane of the present disclosure, the gas in the culture system may be periodically replaced to recover the gas in the culture system containing the produced methane. The gas in the culture system may be replaced at regular time intervals or according to the methane concentration in the culture system. In the case of replacing the gas in the culture system according to the methane concentration in the culture system, the method of producing methane of the present disclosure may further include monitoring the methane concentration in the culture system, determining whether the methane concentration exceeds a set threshold, and replacing the gas in the culture system in a case in which the methane concentration exceeds the set threshold.

### (Control Device)

A control device of the present disclosure is a control device for controlling culture conditions for co-culturing a methanogen and a facultative anaerobic bacterium, the control device including:
a sensor configured to monitor a culture condition of a culture system; and
a controller configured to determine whether the monitored culture condition is outside a set threshold range and control a culture condition to increase oxygen consumption activity of the facultative anaerobic bacterium in a case in which the monitored culture condition is outside the set threshold range.

The control device of the present disclosure can be used in the method of producing methane of the present disclosure. By use of the control device of the present disclosure in the method of producing methane, continuous production of methane with an excellent methane production capability even under oxygen contamination conditions can be achieved. In the control device of the present disclosure, the monitored culture condition may be the oxygen concentration, and the controlled culture condition may be the culture temperature. Alternatively, in the control device of the present disclosure, the monitored culture condition and the controlled culture condition may be the same, and examples thereof include the culture temperature, the pH of the culture medium, the oxidation-reduction potential (ORP) of the culture medium, and the oxygen concentration (for example, the dissolved oxygen (DO) in the culture medium, the concentration of oxygen in the gas phase, or both). Each component of the control device of the present disclosure is described below.

### (Methane Production System)

The methane production system of the present disclosure includes the above-described control device of the present disclosure, a culture tank for co-culturing a methanogen and a facultative anaerobic bacterium, and a mechanism configured to adjust a culture condition for co-culturing the methanogen and the facultative anaerobic bacterium, and adjustment of the culture condition in the mechanism configured to adjust the culture condition is controlled by the control device. By use of the methane production system of the present disclosure, continuous production of methane with an excellent methane production capability even under oxygen contamination conditions can be achieved.

### (Description of Each Component)

Each component of the method of producing methane, the control device, and the methane production system of the present disclosure is described below.

### <Methanogen>

Examples of methanogens include Methanobacterium alcaliphilum, Methanobacterium bryantii, Methanobacterium congolense, Methanobacterium defluvii, Methanobacterium espanolae, Methanobacterium formicicum, Methanobacterium ivanovii, Methanobacterium palustre, Methanobacterium thermaggregans, Methanobacterium uliginosum, Methanobrevibacter acididurans, Methanobrevibacter arboriphilicus, Methanobrevibacter gottschalkii, Methanobrevibacter olleyae, Methanobrevibacter ruminantium, Methanobrevibacter smithii, Methanobrevibacter woesei, Methanobrevibacter wolinii, Methanothermobacter marburgensis, Methanothermobacter thermoautotrophicus, Methanobacterium thermoautotrophicus, Methanothermobacter thermoflexus, Methanothermobacter thermophilics, Methanothermobacter wolfeii, Methanothermus sociabilis, Methanocorpusculum bavaricum, Methanocorpusculum parvum, Methanoculleus chikuoensis, Methanoculleus submarinus, Methanogenium frigidum, Methanogenium liminatans, Methanogenium marinum, Methanomicrobium mobile, Methanocaldococcus jannaschii, Methanococcus aeolicus, Methanococcus maripaludis, Methanococcus vannielii, Methanococcus voltaei, and Methanothermococcus thermolithotrophicus.

### <Facultative Anaerobic Bacterium>

Examples of the facultative anaerobic bacterium include the genus Caldilinea (Caldilinea aerophile and the like), the genus Staphylococcus (Staphylococcus aureus, Staphylococcus epidermidis, Staphylococcu saprophyticus), the genus Corynebacterium (Corynebacterium glutamicum and the like), the genus Listeria (Listeria monocytogenes and the like), and the genus Escherichia (Escherichia coli and the like).

### <Raw Material for Methanogenesis>

Examples of the raw material for methanogenesis include a combination of hydrogen (H₂) and carbon dioxide (CO₂). Examples of methanogenic reactions by methanogens include the following.

CO₂ + 4H₂ → CH₄ + 2H₂O

### <Monitored and Controlled Culture Conditions>

Examples of the culture conditions that are monitored include the oxygen concentration (for example, the amount of dissolved oxygen (DO) in the culture medium), the culture temperature, the pH of the culture medium, the oxidation-reduction potential (ORP) of the culture medium, and the pressure in the reaction vessel.

Examples of the culture conditions that are controlled (adjusted) include the culture temperature, the pH of the culture medium, the oxidation-reduction potential (ORP) of the culture medium, and the pressure in the reaction vessel.

### -Oxygen Concentration-

The oxygen concentration can be monitored using, for example, the amount of dissolved oxygen (DO) in the culture medium, the concentration of oxygen in the gas phase, or both as indicators. Since methanogens are strictly anaerobic microorganisms, and the presence of oxygen (O₂) leads to decreased activity, growth inhibition, and death, the oxygen concentration (for example, the amount of DO in the culture medium, the concentration of oxygen in the gas phase, or both) needs to be as low as possible. The oxygen concentration is monitored, and when it exceeds a preset threshold, the culture conditions are adjusted to increase the activity of the facultative anaerobic bacterium, so that oxygen (O₂) is removed by being consumed by the facultative anaerobic bacterium. Examples of the culture conditions that are adjusted include, for example, the culture temperature. Since the solubility of gas in a liquid decreases with increasing temperature, it is possible to lower the DO level by raising the temperature, but the culture temperature is preferably adjusted so as not to be too high, since a high temperature may lead to death of the bacteria.

The oxygen concentration can be optimized by adjusting the culture temperature using the monitored oxygen concentration as feedback. Such feedback can, for example, be performed using a control device that includes a sensor (for example, a dissolved oxygen sensor or a sensor for oxygen in the gas phase) that monitors the oxygen concentration (for example, the amount of DO in the culture medium, the concentration of oxygen in the gas phase, or both) of the culture system, and a controller that determines whether the monitored oxygen concentration exceeds a set threshold and controls the culture temperature to increase oxygen consumption activity of the facultative anaerobic bacterium in a case in which the monitored oxygen concentration exceeds the set threshold.

### -Culture Temperature-

Each microorganism strain has a specific growth temperature range. Microorganisms cannot grow outside the growth temperature range, and their activity decreases when the culture temperature is outside the optimum growth temperature. If the culture temperature is lower than the growth temperature for a long period of time, it takes time to regain activity. If the culture temperature rises above the growth temperature, the bacteria may die.

In a case in which the optimal growth temperatures of methanogen and the facultative anaerobic bacterium are different, oxygen (O₂) can more actively be removed by performing operations at the optimal growth temperature of the methanogen under normal conditions and changing the culture temperature to the optimal growth temperature of the facultative anaerobic bacterium when the oxygen concentration becomes excessive.

Therefore, the culture temperature may, for example, be adjusted to be the optimal growth temperature of the facultative anaerobic bacterium by using the monitored oxygen concentration as feedback. The culture temperature may also be adjusted by monitoring the culture temperature and using the culture temperature itself as feedback for adjustment to a temperature that is not too high, so as not to lead to the death of the bacteria, for example. In the case of monitoring the culture temperature and using the culture temperature itself as feedback, such feedback may, for example, be performed using a control device that includes a sensor that monitors the culture temperature of the culture system, and a controller that determines whether the monitored culture temperature is outside a set threshold range and controls the culture temperature to increase oxygen consumption activity of the facultative anaerobic bacterium in a case in which the monitored culture temperature is outside the set threshold range. Examples of the controller for controlling the culture temperature include a temperature control device capable of heating or cooling the reaction vessel.

### -pH of Culture Medium-

Each microorganism strain has a specific growth pH range. Microorganisms do not grow outside the pH range that allows growth, and microbial activity decreases when the pH deviates from the optimal growth pH. The pH of the culture medium may, for example, be adjusted by monitoring the pH of the culture medium and using the pH as feedback. When the pH deviates from the optimal growth pH range, alkali or acid may be added to the culture medium to bring the pH back within the range, thereby maintaining good growth conditions for the methanogen and the facultative anaerobic bacterium and achieving continuous production of methane with an excellent methane production capability.

Such feedback may, for example, be performed using a control device that includes a sensor that monitors the pH of the culture medium in the culture system, and a controller that determines whether the monitored pH of the culture medium is outside a set threshold range and controls the pH of the culture medium to increase oxygen consumption activity of the facultative anaerobic bacterium in a case in which the monitored pH of the culture medium is outside the set threshold range. Examples of the controller for controlling the pH of the culture medium include an alkaline solution injection device and an acidic solution injection device.

-Oxidation-Reduction Potential (ORP) of Culture Medium- Methanogens are active in anaerobic conditions and in reducing environments with negative values of oxidation-reduction potential. The value of the oxidation-reduction potential at which a methanogen is active depends on the type of microorganism. In an environment in which facultative anaerobic bacteria are present, methane production is possible with time, but to improve the environment more actively, the oxidation-reduction potential (ORP) of the culture medium is preferably adjusted to achieve good microbial activity. Such adjustment may, for example, be made by adding a reducing agent (sodium sulfide nonahydrate, cysteine hydrochloride monohydrate, or the like) to the culture medium. Since oxygen (O₂) is a factor that increases ORP, possible contamination by oxygen (O₂) may be assumed when ORP increases.

The ORP of the culture medium may, for example, be adjusted by monitoring the oxidation-reduction potential of the culture medium and using the oxidation-reduction potential as feedback. Such feedback may, for example, be performed using a control device that includes a sensor that monitors the oxidation-reduction potential of the culture medium in the culture system, and a controller that determines whether the monitored oxidation-reduction potential of the culture medium is outside a set threshold range and controls the oxidation-reduction potential of the culture medium to increase oxygen consumption activity of the facultative anaerobic bacterium in a case in which the monitored oxidation-reduction potential of the culture medium is outside the set threshold range. Examples of the controller for controlling the oxidation-reduction potential of the culture medium include a reducing agent injection device.

### -Pressure in Reaction Vessel-

Each microorganism strain has a specific growth pressure range. Outside of the growth pressure range, the microorganism will not grow and will die. The pressure in the reaction vessel may be adjusted to the optimum growth pressure of the microorganism by, for example, monitoring the pressure in the reaction vessel and using the pressure as feedback. When the pressure in the reaction vessel pressure falls outside the growth pressure range of the microorganism, the gas phase in the reaction vessel may be discharged through a gas outlet in the reaction vessel to bring the pressure back within the range, for example, thereby maintaining good growth conditions for the methanogen and the facultative anaerobic bacterium and achieving continuous production of methane with an excellent methane production capability.

Such feedback may, for example, be performed using a control device that includes a sensor that monitors the pressure in the reaction vessel in the culture system, and a controller that determines whether the monitored pressure in the reaction vessel is outside a set threshold range and controls the culture temperature to increase oxygen consumption activity of the facultative anaerobic bacterium in a case in which the monitored pressure in the reaction vessel is outside the set threshold range. Examples of the controller for controlling the pressure in the reaction vessel include a gas outlet.

### <Reaction Vessel>

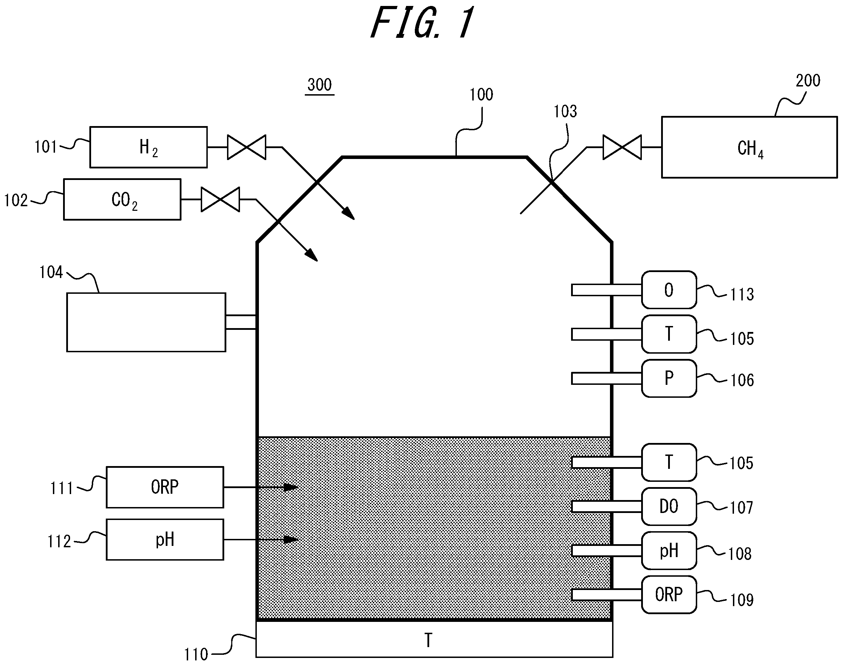

The reaction vessel is not limited to any particular vessel, as long as it can contain the culture medium and the gas phase and can subject the microorganisms to liquid culturing in the culture medium. An example of a reaction vessel 100 and a methane (CH₄) production system 300 that includes the reaction vessel 100 is described below with reference to FIG. 1. The reaction vessel 100 may be connected to a hydrogen (H₂) gas supply 101, a carbon dioxide (CO₂) gas supply 102, a gas outlet 103, a gas analyzer 104, a temperature sensor 105, and a pressure sensor 106 in a gas containment area. The reaction vessel 100 may also be connected to the temperature sensor 105, a dissolved oxygen (DO) sensor 107, a pH sensor 108, an oxidation-reduction potential (ORP) sensor 109, a temperature adjustment mechanism 110, an oxidation-reduction potential (ORP) adjustment mechanism 111, and a pH adjustment mechanism 112 in a culture medium containment area. The reaction vessel 100 may be connected to a methane (CH₄) gas tank 200 via the gas outlet 103. The methane (CH₄) production system 300 may include the reaction vessel 100 and some or all of the above-described components other than the reaction vessel 100.

### EXAMPLES

The present disclosure is described in more detail below using examples, but the technical scope of the present disclosure is not limited by these examples.

### (Example 1)

(1) A culture medium having the same composition as Medium No. 398 (https://www.nite.go.jp/nbrc/catalogue/NBRCMediumDetailServlet?NO=398) listed in the NBRC online catalog was poured into the reaction vessel. (2) The vessel was sealed and filled, as the gas phase portion, with an H₂/CO₂ mixture (mixture ratio H₂:CO₂ = 80:20 vol.%), which is a reaction gas. (3) A microorganism group including a hydrogenotrophic methanogen and a facultative anaerobic bacterium were injected into the culture medium. (4) Air was added to the gas phase portion so that the oxygen (O₂) content was 1 vol.% at the start of culturing. (5) The vessel was pressurized with the reaction gas until reaching the set pressure for the start of culturing. (6) Culturing was conducted under a predetermined constant temperature condition. (7) After confirming that the supplied CO₂ was converted to methane, the produced methane gas was replaced with H₂/CO₂, i.e., the reaction gas, and air was added to maintain the same conditions as in (4) above. Pressurization to the set pressure with the reaction gas was then performed, and culturing was continued.

The culture medium contained a hydrogenotrophic methanogen of the genus Methanothermobacter and a facultative anaerobic bacterium of the genus Caldilinea.

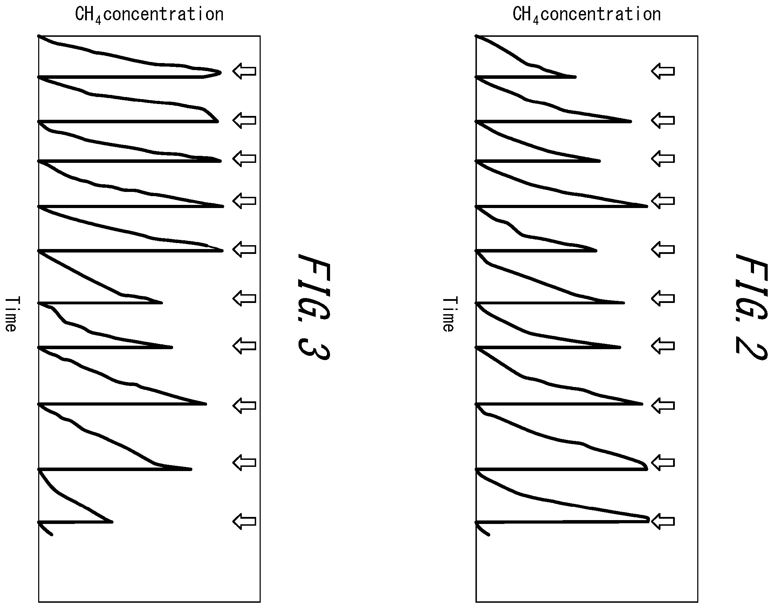

The results of the change over time in the methane concentration (an indicator of the amount of methane produced) in the reaction vessel are illustrated in FIG. 2. The arrows indicate the points at which the produced methane gas was replaced by the H₂/CO₂ mixture, i.e., the reaction gas. The results in FIG. 2 indicate that good methane production was maintained even in the presence of O₂, which has the characteristic of inhibiting the activity of hydrogenotrophic methanogens.

### (Reference Example 1)

Methane was produced by standard culturing using a methanogen of the genus Methanothermobacter alone. Specifically, culturing was conducted in the same manner as in Example 1, except that the culture medium that was used contained a methanogen of the genus Methanothermobacter alone and did not contain facultative anaerobic bacteria, and no air was added to the gas phase portion during culturing.

The results of the change over time in the methane concentration (an indicator of the amount of methane produced) in the reaction vessel are illustrated in FIG. 3. The arrows indicate the points at which the produced methane gas was replaced by the H₂/CO₂ mixture, i.e., the reaction gas. The results in FIG. 3 indicate that good methane production was maintained even though the culture medium did not contain facultative anaerobic bacteria, since O₂, which has the characteristic of inhibiting the activity of hydrogenotrophic methanogens, was not added.

### (Comparative Example 1)

Culturing was conducted in the same manner as in Example 1, except that the culture medium that was used contained a hydrogenotrophic methanogen of the genus Methanothermobacter alone and did not contain facultative anaerobic bacteria.

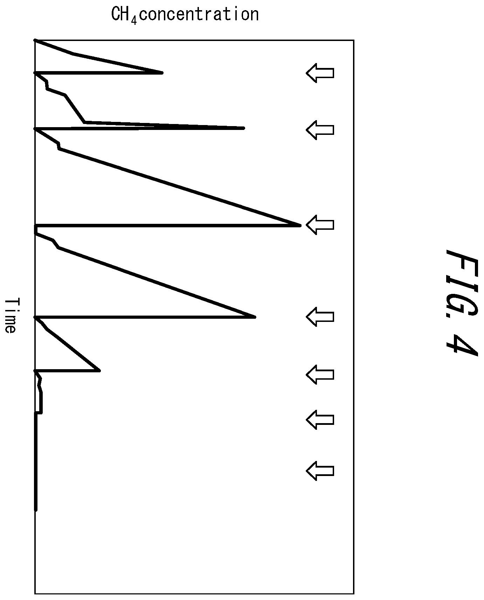

The results of the change over time in the methane concentration (an indicator of the amount of methane produced) in the reaction vessel are illustrated in FIG. 4. The arrows indicate the points at which the produced methane gas was replaced by the H₂/CO₂ mixture, i.e., the reaction gas. The results in FIG. 4 indicate that the non-inclusion of a facultative anaerobic bacterium caused the added O₂ to accumulate without being consumed, thereby degrading the growth condition of the hydrogenotrophic methanogen. The methane production capability then decreased as the culture time progressed, and eventually methane production almost ceased.

### (Results)

In Example 1, which follows the method of producing methane of the present disclosure, good methane production continued to the same degree as in Reference Example 1, which illustrates methane production by standard culturing using a hydrogenotrophic methanogenic bacterium, despite the presence in Example 1 of oxygen (O₂), which has the characteristic of inhibiting the activity of hydrogenotrophic methanogens. On the other hand, in Comparative Example 1, which was cultured with a culture medium that did not contain facultative anaerobic bacteria, the methane production capability decreased as the culture time progressed, and eventually methane production almost ceased. These results indicate that by following the method of producing methane of the present disclosure, methane can be produced well even in the presence of oxygen, which has the characteristic of inhibiting the activity of hydrogenotrophic methanogens.

**[Table 1]**

| | | Example 1 | Reference Example 1 | Comparative Example 1 |
|---|---|---|---|---|
| Culture medium | Methanogen | + | + | + |
| | Facultative anaerobic bacterium | + | - | - |
| O₂ addition | | + | - | + |
| Methane production capability | | Good | Good | Poor |

### INDUSTRIAL APPLICABILITY

According to the present disclosure, a method of producing methane using a methanogen, as well as a control device and a methane production system useable in this method, that achieve an excellent methane production capability continuously even under oxygen (O₂) contamination conditions can be provided.

### REFERENCE SIGNS LIST

- 100: Reaction vessel
- 101: Hydrogen (H₂) gas
- 102: Carbon dioxide (CO₂) gas supply
- 103: Gas outlet
- 104: Gas analyzer
- 105: Temperature sensor
- 106: Pressure sensor
- 107: Dissolved oxygen (DO) sensor
- 108: pH sensor
- 109: Oxidation-reduction potential (ORP) sensor
- 110: Temperature adjustment mechanism
- 111: Oxidation-reduction potential (ORP) adjustment mechanism
- 112: pH adjustment mechanism
- 113: Oxygen sensor
- 200: Methane (CH₄) gas tank
- 300: Methane (CH₄) production system

## Claims

1. A method of producing methane, comprising co-culturing a methanogen and a facultative anaerobic bacterium to produce methane.

2. The method of producing methane according to claim 1, further comprising:
monitoring a culture condition of a culture system;
determining whether the monitored culture condition is outside a set threshold range; and
adjusting a culture condition to increase oxygen consumption activity of the facultative anaerobic bacterium in a case in which the monitored culture condition is outside the set threshold range.

3. The method of producing methane according to claim 2, wherein the monitored culture condition is oxygen concentration, and a controlled culture condition is culture temperature.

4. The method of producing methane according to claim 2, wherein the monitored culture condition and a controlled culture condition are culture temperature, pH of a culture medium, oxidation-reduction potential of the culture medium, or pressure in a reaction vessel.

5. A control device for controlling culture conditions for co-culturing a methanogen and a facultative anaerobic bacterium, the control device comprising:
a sensor configured to monitor a culture condition of a culture system; and
a controller configured to determine whether the monitored culture condition is outside a set threshold range and control a culture condition to increase oxygen consumption activity of the facultative anaerobic bacterium in a case in which the monitored culture condition is outside the set threshold range.

6. The control device according to claim 5, wherein the monitored culture condition is oxygen concentration, and the controlled culture condition is culture temperature.

7. The control device according to claim 5, wherein the monitored culture condition and the controlled culture condition are culture temperature, pH of a culture medium, oxidation-reduction potential of the culture medium, or pressure in a reaction vessel.

8. A methane production system comprising:
the control device according to any one of claims 5 to 7;
a reaction vessel for co-culturing a methanogen and a facultative anaerobic bacterium; and
a mechanism configured to adjust a culture condition for co-culturing the methanogen and the facultative anaerobic bacterium, wherein
adjustment of the culture condition in the mechanism configured to adjust the culture condition is controlled by the control device.
